Europäisches Patentamt

European Patent Office

Office européen des brevets

(19)

(11) Veröffentlichungsnummer : **0 024 534**
**B1**

(12) **EUROPÄISCHE PATENTSCHRIFT**

(45) Veröffentlichungstag der Patentschrift :
26.01.83

(21) Anmeldenummer : 80104186.4

(22) Anmeldetag : 17.07.80

(51) Int. Cl.³ : **C 07 C 69/54**, C 07 C 67/62,
**C 09 K 15/30**

(54) **Gegen vorzeitige Polymerisation stabilisierte Monomere Acrylsäureester und Verfahren zu deren Stabilisierung.**

(30) Priorität : 03.08.79 DE 2931553

(43) Veröffentlichungstag der Anmeldung :
11.03.81 Patentblatt 81/10

(45) Bekanntmachung des Hinweises auf die Patenterteilung : 26.01.83 Patentblatt 83/04

(84) Benannte Vertragsstaaten :
DE FR GB

(56) Entgegenhaltungen :
EP A 0 003 045
AT B 340 377

(73) Patentinhaber : BASF Aktiengesellschaft
Carl-Bosch-Strasse 38
D-6700 Ludwigshafen (DE)

(72) Erfinder : Schropp, Wilhelm Karl, Dr.
Im Langgewann 67
D-6940 Weinheim (DE)

**0 024 534**

Gegen vorzeitige Polymerisation stabilisierte monomere Acrylsäureester und Verfahren zu deren Stabilisierung

Die Erfindung betrifft die Verhinderung vorzeitiger Polymerisation bei der Herstellung, beim Transport und bei der Lagerung von monomeren Acrylsäureestern bzw. solche enthaltende Zubereitungen durch Zugabe von Phenothiazin und para-Nitrosophenol.

Erhebliche Mengen an Acrylsäureestern, wie besonders die Methyl-, Aethyl-, Butyl- und 2-Aethylhexylester, dienen für die Herstellung von Polymerisaten und Mischpolymerisaten. Bei ihrer Herstellung, Lagerung und bei ihrem Transport muß eine vorzeitige Polymerisation verhindert werden. Kommt es zu einer vorzeitigen Polymerisation, werden Reaktionsgefäße, Rohrleitungen, Destillationskolonnen, Kondensatoren, Lager behälter etc. durch zähe bis klebrige, schwer zu entfernende Polymerisate verschmutzt oder verstopft, so daß die betroffene Anlage ganz oder teilweise stillgelegt und gereinigt werden muß. Die Behebung derartiger Betriebsstörungen ist zeitraubend und häufig mit hohen Kosten verbunden, insbesondere dann, wenn das gebildete Polymerisat nicht mehr gelöst, sondern mechanisch entfernt werden muß. Besonders anfällig dür die Bildung von Polymerisat sind die für die Reinigung der Acrylester benutzten Destillationskolonnen.

Weiterhin kann es bei der Lagerung von Acrylsäureestern durch die bei der vorzeitigen Polymerisation freiwerdende Reaktionswärme zu gefährlichen Betriebszuständen kommen.

Für die Stabilisierung von Estern der Acrylsäure sind daher eine Vielzahl von Stoffen vorgeschlagen worden, vgl. z.B. « Ullmanns Encyclopädie der technischen Chemie », 3. Auflage, Band 14 (1963), Seite 116. Am häufigsten werden Phenole, besonders Hydrochinon und Hydrochinonmonomethyläther, sowie aliphatische und aromatische Amine verwendet. Beispiele für Amine sind para-Phenylendiamin und seine Derivate, sowie Phenothiazin, das speziell bei Acrylsäure und Acrylsäureestern mit Erfolg eingesetzt wird. Auch Derivate des Phenols, wie para-Nitrosophenol wurden gelegentlich als Stabilisatoren für Acrylester genannt (z.B. GB-PS 1 064 845).

Es wurde nun überraschend gefunden, daß man monomere Acrylsäureester 1 bis 8 C-Atome enthaltender Alkanole mit einem Gemisch aus Phenothiazin und para-Nitroso-phenol besonders wirksam gegen vorzeitige Polymerisation stabilisieren kann. Das erfindungsgemäße Stabilisatorgemisch ist wesentlich wirksamer als die Komponenten für sich und wirksamer als die Summe der Wirksamkeiten der Komponenten.

Man stabilisiert, wie an sich üblich, meist derart, daß man die vorgesehene Menge der Stabilisatoren getrennt oder als Gemisch in den Acrylestern unter Rühren auflöst. Besonders vorteilhaft ist es, konzentrierte Lösungen in den Monomeren einzusetzen und diese den restlichen Monomeren in den Reaktionsgefäßen, Destillationskolonnen, Kühlern u.a. zuzugeben. Die Löslichkeit von Phenothiazin (I) und para-Nitrosophenol (II) in Acrylestern 1 bis 8 C-Atome aufweisender Alkanole ist ausreichend hoch, um in dieser Weise vorzugehen.

Die Wirkung der Stabilisatorgemische ist, wie an sich üblich, konzentrations- und temperaturabhängig, d.h. die Zeitspanne bis zum Beginn der Polymerisation ist umso länger, je mehr Stabilisatorgemisch zugegeben wird und verkürzt sich bei höheren Temperaturen. Konzentrationen an Stabilisatorgemisch von 1 ppm, bezogen auf das Gewicht des Monomeren, sind schon wirksam. Üblicherweise werden für die meisten Anwendungen Konzentrationen von 5 bis 100 ppm angewandt, wobei mitunter auch höhere Konzentrationen bis zu 1 000 ppm gewählt werden, insbesondere dann, wenn bei höheren Temperaturen gearbeitet werden muß. Obgleich noch höhere Dosierungen ebenfalls wirksam sind, werden diese aus wirtschaftlichen Gründen vermieden. Meist liegen somit die Mengen an Stabilisatorgemisch zwischen 1 und 100 ppm, bezogen auf die Monomeren. Eine verbesserte Stabilisierung monomerer Acrylsäureester wird beobachtet, wenn die Menge von (I) 80 bis 20 Gewichtsprozent des Gemisches aus (I) und (II) beträgt. Im allgemeinen liegt der Gewichtsanteil von (I) zwischen 70 und 30 Gewichtsprozent, und Mengen von 60 bis 40 Gewichtsprozent werden bevorzugt. Oft sind annähernd äquimolare Gewichtsmengen der Komponenten besonders wirksam.

Die erfindungsgemäßen Stabilisatorgemische verlängern die Induktionsperiode vor dem Anlaufen der Polymerisation von Acrylestern. Die Induktionsperiode, d.h. die Zeitspanne vom Beginn der Untersuchung bis zum Eintritt der Polymerisation, kann z.B. in einfacher Weise gemessen werden, indem man die Testgemische in Proberöhrchen einschmilzt, in einen Thermostaten mit guter Temperaturkonstanz einbringt und durch gelegentliches Wenden der Röhrchen feststellt, ob die Probe noch dünnflüssig oder durch Polymerisation fest geworden ist.

Die Bewertung der Stabilisatorgemische des erfindungsgemäßen Verfahrens in den nachfolgenden Beispielen wurde so durchgeführt, wobei von jeder gewählten Stabilisatorkonzentration mindestens vier Proben angesetzt wurden. Die Badtemperatur betrug durchweg $80 \pm 0,5\,°C$.

Alle verwendeten Monomeren waren zur Abtrennung des Lagerstabilisators (= Hydrochinonmonomethyläther) durch Vakuum-Destillation gereinigt; ihre Stabilisatorrestgehalte betrugen weniger als 0,2 ppm.

Als Stabilisatoren wurden handelsübliche (I) (Reinheit mind. 99 %) und handelsübliches (II) (Reinheit ca. 83 %, Rest Wasser) eingesetzt.

Zur Herstellung der Probelösungen wurden die Stabilisatoren abgezwogen und in 1/10 der erforderlichen Acrylatmenge gelöst. Nach einstündigem Rühren wurde filtriert und verdünnt.

2

**0 024 534**

Beispiel 1

Stabilisierung von Methylacrylat

Tabelle 1

| Menge an (I) in ppm | Menge an (II) in ppm | Zeitspanne bis zur Polymerisation (Stunden) |
|---|---|---|
| 4 | 1 | 12,5 |
| 3 | 2 | 18,5 |
| 2 | 3 | 15,0 |
| 1 | 4 | 6,1 |
| 30 | 20 | 326 |
| 20 | 30 | 309 |

Während Stabilisator-freies Methylacrylat nur 1,9 Stunden stabil ist, erhöht sich die Stabilität durch Zugabe von 5 ppm (I) bzw. 5 ppm (II) auf 6 bzw. 3,2 Stunden. Weit übertroffen werden diese Ergebnisse, wenn — unter Beibehaltung der Gesamtkonzentration — Kombinationen aus den beiden Stabilisatoren eingesetzt werden. Mit 3 ppm (I) und 2 ppm (II) wird sogar eine mehr als dreimal so hohe Stabilität erzielt wie mit 5 ppm (I).

Der gleiche Effekt tritt auch bei einer Gesamtkonzentration von 50 ppm auf, wie aus dem unteren Teil von Tabelle 1 ersichtlich ist, wobei die Induktionsperiode bei 50 ppm (I) 130 Stunden, bei 50 ppm (II) 68 Stunden beträgt.

Beispiel 2

Stabilisierung von Äthylacrylat

Praktisch Stabilisator-freies Äthylacrylat hat eine Induktionsperiode von 6 Stunden. Bei einem Gehalt von 10 ppm (I) und 10 ppm (II) beträgt die Induktionsperiode 260 Stunden. Dagegen führt ein Gehalt von 20 ppm (I) zu einer Induktionsperiode von nur 129 Stunden, ein Gehalt von 20 ppm (II) von nur 25 Stunden.

Beispiel 3

Stabilisierung von n-Butylacrylat

| Mengen an (I) in ppm | Mengen an (II) in ppm | Zeit bis zur Polymerisation (Stunden) |
|---|---|---|
| 10 | 10 | 514 |
| 8 | 12 | 534 |
| 6 | 14 | 579 |
| 2 | 18 | 351 |

Zum Vergleich beträgt die Induktionsperiode bei praktisch Stabilisator-freiem Butylacrylat 10 Stunden, bei einem Gehalt von nur 20 ppm (I) 251 Stunden und bei einem Gehalt von nur 20 ppm (II) nur 49 Stunden

Beispiel 4

Stabilisierung von 2-Äthylhexylacrylat

Praktisch stabilisatorfreies 2-Äthylhexylacrylat hat eine Induktionsperiode von 6 Stunden. Erfindungsgemäß stabilisiertes 2-Äthylhexylacrylat mit einem Gehalt von 2,5 ppm (I) und 2,5 ppm (II) polymerisiert nach 69 Stunden. Zum Vergleich beträgt die Induktionsperiode bei einem Gehalt von ausschließlich 5 ppm (I) 37 Stunden, bei einem Gehalt von ausschließlich 5 ppm (II) 20,5 Stunden.

**Ansprüche**

1. Verfahren zur Stabilisierung monomerer Acrylsäureester 1 bis 8 C-Atome enthaltender Alkanole

3

**0 024 534**

gegen vorzeitige Polymerisation durch ein p-Nitrosophenol enthaltendes Stabilisatorgemisch, dadurch gekennzeichnet, daß man den Acrylsäureestern 1 bis 1 000 ppm eines Gemisches aus 80 bis 20 Gew.% Phenothiazin und 20 bis 80 Gew.%, bezogen auf das Stabilisatorgemisch, p-Nitrosophenol zusetzt.

2. Acrylsäureester 1 bis 8 C-Atome enthaltender Alkanole, die als Stabilisatoren gegen vorzeitige Polymerisation 1 bis 1 000 ppm eines Gemisches aus 80 bis 20 Gew.% Phenothiazin und 20 bis 80 Gew.%, bezogen auf das Stabilisatorgemisch, p-Nitrosophenol enthalten.

## Claims

1. A process for stabilizing monomeric esters of acrylic acid with alkanols of 1 to 8 carbon atoms against premature polymerization by means of a mixture of phenothiazine and para-nitrosophenol, wherein from 1 to 1 000 ppm of a mixture of from 80 to 20 percent by weight of phenothiazine and from 20 to 80 percent by weight of p-nitrosophenol, the percentages being based on the stabilizer mixture, are added to the acrylic acid ester.

2. An ester of acrylic acid with an alkanol of 1 to 8 carbon atoms, which ester contains as a stabilizer against premature polymerization from 1 to 1 000 ppm of a mixture of from 80 to 20 percent by weight of phenothiazine and from 20 to 80 percent by weight of p-nitrosophenol, the percentages being based on the stabilizer mixture.

## Revendications

1. Procédé de stabilisation d'esters acryliques monomères d'alcanols contenant 1 à 8 atomes C, contre la polymérisation prématurée par un mélange stabilisateur contenant du p-nitrosophénol, caractérisé par le fait qu'on ajoute aux esters acryliques 1 à 1 000 ppm d'un mélange de 80 à 20 % en poids de phénothiazine et 20 à 80 % en poids, rapportés au mélange stabilisant, de p-nitrosophénol.

2. Esters acryliques d'alcanols contenant 1 à 8 atomes C qui contiennent, comme stabilisant contre une polymérisation prématurée, 1-1 000 ppm d'un mélange de 80 à 20 % en poids de phénothiazine et 20 à 80 % en poids, rapportés au mélange stabilisant, de p-nitrosophénol.

4